# EUROPEAN PATENT APPLICATION

(11) **EP 2 312 509 A1**
(43) Date of publication of application: **20.04.2011**
(21) Application number: 10380096.7
(22) Date of filing: 23.07.2010
(51) Int. Cl.: G06Q 10/00

(54) **Electronic system for the management of the replenishment and filling of plant stores in hospitals**

(30) Priority: 14.10.2009 ES 200930847
(71) Applicant: Grifols, S.A., 08022 Barcelona (ES)
(72) Inventor: Sanchez Maulini, Manuel, 08970 Sant Joan Despi (Barcelona) (ES); Blanquer Torre, Roberto, 08023 Barcelona (ES)
(74) Representative: Durán Moya, Luis-Alfonso

(57) **Abstract**

System for the replenishment and filling of stores which comprises at least one full function device (FFD) capable of bidirectional wireless communication with at least one RFD and is capable of data processing, and at least one reduced function device (RFD) capable of bidirectional wireless communication, characterised in that said RFD comprises a push-button, an RF transceiver, an indicator device and a battery and also emits a restocking request signal to the FFD when said button is pressed.

## Description

The present invention relates to an electronic system for the management of the replenishment and filling of stores and storage devices (mobile or fixed). Although the present invention is particularly indicated for hospital environments, its use is not necessarily limited to that sector.

In present health facilities the system for supplying materials or medicines to health professionals may be centralised or decentralised. In the first case, which is fairly effective for low-capacity medical centres, a central pharmacy is provided where every time a medicine or equipment is required, the health professional must go to the pharmacy and ask for the material to be supplied. In the second case, due to the size of the facility and/or the amount of material to be supplied, it is more convenient to provide additional lower-capacity storage devices (fixed or mobile) with the supplies used frequently by health staff to avoid all staff having to go to a single pharmacy, which would be inefficient. In the second case, which is the one with which the present invention is concerned, it is important to have a fast and effective system to control the supply of material and a system that provides a simple way of asking for restocking.

In the prior art, various earlier documents are known which propose a solution for this type of storage. For example document EP 1908002 by Miller et al. proposes the use of RFID labels associated with the medicine contained in each compartment of a mobile store in a hospital. Restocking occurs by reading the bar code of the medicine and/or product to be replenished in a remote device, and a signal is emitted by electromagnetic transmission using RFID methodology to all the RFID labels relating to said medicine. Two of the main drawbacks of this device are:
1. Because of the large amount of wireless equipment currently used in medical work, said storage system usually has to operate on a band which is also assigned to other applications and the system implemented must therefore be able to operate at high noise and interference levels. Using RFID, this problem can only be solved by increasing the transmission power and/or the number of antennae. Consequently, RFID technology is not totally suited to this type of application since transmissions made by the antenna (or the plurality of antennae) must use considerable power.
2. RFID labels require the existence of reception to begin transmission (backscatter). Consequently, the device of EP1908002 does not have autonomous means of requesting restocking. If the system to be implemented must indicate the need for restocking, it has to have a continuously transmitting antenna to determine exactly when a restocking requirement arises. This continuous transmission requires unnecessary use of power and, in addition, generates noise which affects the proper functioning of other devices on the same frequency band.

It must be borne in mind that the object of EP 1908002 is not the prior identification of the storage devices that need restocking, (there is therefore no restocking request), EP 1908002 simply notifies which storage devices have a particular product. An operator must then check manually whether or not said storage device needs restocking.

Document US 2004 0225409 by Duncan discloses the use of RFID labels in a medicine restocking method for a pharmacy. To perform this task an RFID label is placed in each drawer of a centralised cupboard containing medicines. The aim of this document is to monitor the location of each of the drawers by means of the labels for the purpose of automatic reconfiguration, and to switch on a luminous indicator on each one, which emits a particular signal if restocking is required (said luminous indicator being, in addition, independent of the label). In document US 2004 0225409, the need for restocking is determined automatically by the inventory system and there is therefore no physical check that it is correct.

An object of the present invention is to disclose a system which solves the above problems and, in addition, allows storage systems to be decentralised, principally in the hospital environment, improves inventory control and makes the replenishment of each satellite store (fixed or mobile) more efficient, thus saving time and space, and obtaining a simple, economical solution that can be applied to the existing hospital infrastructure, thus reducing implementation costs.

By using LR-WPAN networks or similar technologies (such as low speed Bluetooth, Zigbee or those described in the IEEE 802.15.4 or IEEE 1902.1 standards) known as low-rate data transmission wireless personal area networks, particular embodiments of the present invention aim to use far simpler, more compact devices, with a lower cost and lower consumption to obtain better communication between the RF labels and the zone controllers.

More specifically, the present invention describes a replenishment management and filling system for stores which comprises:
- at least one full function device (FFD) with bidirectional wireless communication capacity with at least one RFD device and data processing capacity, and
- at least one reduced function device (RFD) with bidirectional wireless communication capacity;
in which said RFD comprises a push-button, an RF transceiver, an indicator device and a battery and also emits a restocking request signal to the FFD when said button is pushed.

In the telecommunications, electronics and related technical sectors, RFDs are known as reduced function devices and can only operate as a slave in a star configuration. They have the advantage of low energy consumption as a result of low data processing and storage capacity. An RFD can only communicate with a full function device (FFD). In a preferred application of the present invention, the low consumption device (RFD) is placed in each of the mobile storage devices, as its low consumption makes it suitable for operating via batteries. The FFD is a device with higher data processing and storage capacity and can operate as a network coordinator, performing most of the communication work. In the present invention, the FFD is responsible for coordinating a network of RFDs and may be a fixed unit capable of RF transmission, RF reception, data storage and processing and may be located to suit the requirements of the customer.

Among other problems, the present invention solves those relating to energy consumption and operation that exist in the prior art described above. The system according to the present invention allows low cost and low consumption units to be used and material restocking requests to be made via a simple operation.

The present invention preferably uses the IEEE 802.15.4 standard although it may also use other types of network, such as those mentioned above.

The push-button of the present invention may be located in a fixed or mobile storage device to transmit the restocking requirement to a zone controller. In these circumstances, communication preferably using the IEEE 802.15.4 standard is more efficient because constant transmission (as with RFID) is not necessary. On the contrary, the system only uses transmission devices when restocking is requested or when, in the opposite case, the labels are to be switched on, indicating the need for restocking. In addition, if there is no need for signal transmission or reception (no change in the state of the RFDs or FFDs), the system can switch to low consumption mode (sleep mode), which represents a considerable energy saving as mentioned earlier. In addition, an advantage of the present invention over what was known previously (for example in EP 1908002), is that, using this device, the labels that require restocking can be identified and only those that need to be restocked are illuminated.

With regard to the proposal of US 2004 0225409, in addition to the advantages mentioned above, the present invention allows the use of RFDs with their own battery, which provides enough energy to notify the FFD. Thus, the system does not depend on the power supply or the initial configuration of a computer system indicating the location and state of the label, which allows the storage device to be moved. Nor is it necessary to reconfigure the program every time the storage device associated with a label is moved. Said reconfiguration represents a major and unnecessary computer cost for the particular application of mobile storage devices in hospitals, which constantly change location.

One of the main advantages of the system of this invention, in addition to its low cost, is its low complexity, combined with the use of low consumption electronics, making it compatible with the standard concerning the use of radiofrequency equipment in hospital environments. For transmissions in health environments from devices in the ICM5 band (from 2.4 to 2.5 GHz), said standard recommends limiting the maximum transmission power to 500 mW.

Another object of the present invention is to disclose a system that allows existing storage devices to be used in order to reduce the cost of implementation. To achieve this object, the present invention comprises an RFD, which is preferably low cost, has small dimensions and low power consumption, and is located in the storage sites where the system is to be implemented. The function of said RFD is to emit a signal to the FFD when restocking is required and preferably notify the end user when the FFD emits a signal with its code.

Another object of the present invention is to disclose a bidirectional wireless communication device in replenishing and management systems for stores, characterised in that it comprises an RF transceiver, a indicator device, a switch and a battery for network communication described in the IEEE 802.15 or IEEE 1902.1 standard. The device is an RFD since, because it is a device that operates on a battery supply, it is advisable for its consumption to be as low as possible. Said energy saving is achieved by reducing the functionality of the device to the minimum possible.

For a better understanding of the invention, the accompanying drawings of an embodiment of the present invention are given as an explanatory but not limiting example.
Fig. 1 shows a mobile storage device, and
Fig. 2 shows the complete replenishment management and filling system.

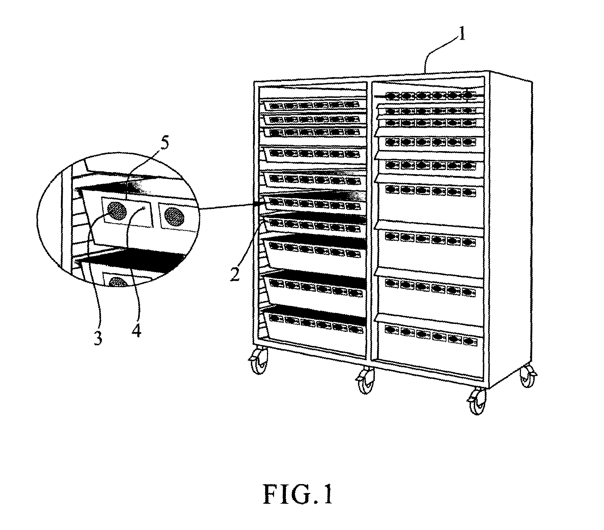

Fig. 1 shows in detail the storage device in which a mobile storage device -1- can be seen which preferably will already be present in the facilities of the customer, which has drawers -2- for storing medicines or any other type of medical equipment. A detail is also shown of the RFD -5- which has a push-button -3- of which the main function is to request restocking and an LED -4- to notify the operator visually.

It is important to remember that in this embodiment in particular, an RFD -5- is located in each drawer -2- of each mobile storage device -1-. Said RFD -5- can transmit a signal by radiofrequency to a zone controller (not shown) indicating its code, which is unique to the RFD. Said zone controller (not shown) with the code received from the RFD -5- knows what material to supply to the storage device corresponding to the RFD -5-.

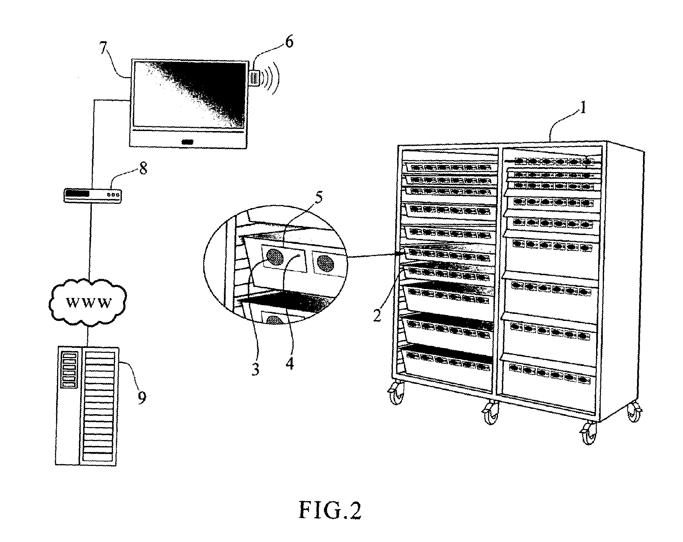

Fig. 2 shows the complete system disclosed by the present invention (replenishment management and filling) in which can be seen a mobile storage device -1-, drawers -2- for storing medicines or any type of medical material and RFDs -5- which preferably comprise a push-button -3- and an LED -4-. Also shown is the FFD or zone controller -7- with its respective radiofrequency transmission antenna -6-, a router (optional) -8- for connecting to the data network of the customer facilities and a server -9- which supports an inventory application and thus controls the amount of material located in each storage device -1-, as well as keeping a record of the restocking requests made by each storage device -1-.

In a preferred embodiment the system communicates using the IEEE 802.15.4 standard, as it is more efficient with regard to noise reduction than that described by the ISO-18000-4 standard (RFID standard) and is therefore inherently less susceptible to interference than earlier known devices. In a preferred embodiment of the present invention, the system communicates using the IEEE 802.15.4 protocol, and consequently uses the direct sequence spread spectrum (DSSS) for data transmission and reception whereas ISO-18000-4 uses the frequency hopping spread spectrum (FHSS).

There is a belief accepted in the prior art that devices using DSSS (for example IEEE 802.15.4) consume more energy than those that use FHSS (for example RFID), but the improvement in eliminating noise and parasitic interference when using DSSS is significant compared with other extended spectrum techniques. However, surprisingly, the use of the IEEE 802.15.4 standard by the present invention involves considerable energy savings in other areas (such as the use of fewer antennae -6-). Thus, the use of DSSS improves transmission conditions compared with the prior art. It is also advisable to use said DSSS technique since the present invention operates preferably in a high activity band such as that between 2.4 and 2.5 GHz.

In a particularly preferred embodiment, the system comprises in particular:
- at least one RFD -5-,
- at least one zone controller (FFD) -7-,
- a replenishment management application, and
- a main server -9-.

An RFD -5-, identified by a unique code, with an LED indicator -4- and a push-button -3-, is located in each of the storage drawers -2- of the different medicines in mobile stores -1- found in hospitals.

There is a zone controller -7- preferably comprising an RF transceiver, a bar code reader and a replenishment management application plus communication, preferably by cable, with the main server -9-.

The RFD -5- also has the ability to receive information sent from the zone controller -7- and can emit a notification signal, preferably luminous, so that it can be easily located. Preferably, the zone controller -7-, as explained above, can store the code for each RFD -5- and the material contained in the storage device -2- associated with each button and also can preferably read each code relating to the products stored in each storage device. Thus when the product code is input, the zone controller -7- has all the necessary information on which storage device -2- has requested restocking and can therefore emit its code.

If restocking is required, an operator presses the button located on the RFD -5- of the storage device -2-, the RFD -5- transmits the code it has been assigned to the zone controller -7- and, using the replenishment management application, the zone controller -7- stores the code of the RFD -5- which is requesting restocking, identifies the product corresponding to said drawer -2- and sends a restocking request to the main server -9- via the cable network of the hospital.

When one of the storage devices -2- needs supplying, the code of the product to be supplied is input to the zone controller -7-. The controller holds data on where to supply the product and sends the code to the RFD or RFDs - 5- for each storage device -2-. Said RFD -5- receives the signal and if said signal corresponds to its code, it activates the visual notification signal, preferably an LED -4-, so that the operator can easily determine its location.

In a particularly preferred embodiment, when the requested material is to be supplied, a bar code reader (not shown) located in the zone controller -7- is used to identify the product and using the replenishment management application, the system carries out a search to determine which of the storage devices -2- corresponds to the code read by the zone controller -7-. Next, it emits only the code of the RFDs -5- that need restocking. The RFD -5- captures the signal, processes it and if the code received is its own, emits a signal to the LED -4- so that it lights up making the task of the operator responsible for replenishment much easier and more efficient.

Although the invention has been described in relation to examples of preferred embodiments, these should not be considered as limiting the invention which will be defined by the following claims.

## Claims

1. System for the management of the replenishment and filling of stores which comprises:
- at least one full function device (FFD) with the capacity for bidirectional wireless communication with at least one RFD and data processing capacity, and
- at least one reduced function device (RFD) with bidirectional wireless communication capacity;
in which said RFD comprises a push-button, an RF transceiver, a indicator device and a battery and also emits a restocking request signal to the FFD when said button is pressed, the RFD being the device located in the storage location and the FFD being remote from said storage location.

2. System according to claim 1, **characterised in that** it also comprises a main server for centralised inventory control.

3. System according to any one of the preceding claims, **characterised in that** said communication between the RFDs and FFDs occurs in a low-rate wireless personal area network (LR-WPAN).

4. System according to any one of the preceding claims, **characterised in that** said communication between the RFDs and FFDs takes place in a wireless network of the type described in the IEEE 802.15 or IEEE 1902.1 standard.

5. System according to any one of the preceding claims, **characterised in that** the FFD comprises a bar code reader.

6. System according to claim 5, **characterised in that** the code read by said bar code reader of said FFD is used by said FFD to identify the RFD or RFDs with which it will communicate.

7. System according to any one of the preceding claims, **characterised in that** when a code is introduced in the FFD, said FFD processes the data, identifies the RFD or RFDs with which it should communicate and emits a command to the previously identified RFDs to activate the indicator device.

8. System according to any one of the preceding claims, **characterised in that** said RFD and said FFD have an energy saving mode.

9. System according to any one of the preceding claims, **characterised in that** said indicator device is an optical indicator device.

10. System according to any one of the preceding claims, **characterised in that** said FFDs are fixed units and the RFDs are mobile devices located in the area covered by the FFDs.

11. System according to any one of the preceding claims, **characterised in that** said FFDs and RFDs transmit and receive data using the direct sequence spread spectrum (DSSS).

12. System according to any one of the preceding claims, **characterised in that** said FFDs and RFDs transmit and receive data using the band between 2.4 and 2.5 GHz.

13. System according to any one of the preceding claims, **characterised in that** it uses transmission power equal to or less than 500 mW.

14. System according to any one of the preceding claims, **characterised in that** said mobile storage devices are mobile stores for storing medical material in hospitals.

15. System according to claim 14, **characterised in that** said RFD is located in each of the drawers of said mobile stores.

16. Device for bidirectional wireless communication in management and filling systems for stores which consists of an RF transceiver, a indicator device, a switch and a battery **characterised in that**, via communication in networks described in the IEEE 802.15 or IEEE 1902.1 standard, an FFD can be asked for notification.

17. Device according to claim 16, **characterised in that** said indicator device is a light emitting diode (LED).

18. Device according to either claim 16 or claim 17, **characterised in that** said transceiver comprises means of transmitting and receiving in the direct sequence spread spectrum (DSSS).

19. Device according to claims 16 to 18, **characterised in that** said transceiver comprises means of transmitting and receiving in the 2.4 to 2.5 GHz band.

20. Device according to claims 16 to 19, **characterised in that** said transceiver has transmission power equal to or less than 500 mW.
